# EUROPEAN PATENT SPECIFICATION

(11) **EP 3 317 242 B1**
(45) Date of publication and mention of the grant of the patent: **29.07.2020**
(21) Application number: 16732640.4
(22) Date of filing: 28.06.2016
(51) Int. Cl.: C07C 29/132, C07C 31/20

(54) **PROCESS FOR THE PREPARATION OF GLYCOLS**
VERFAHREN ZUR HERSTELLUNG VON GLYCOLEN
PROCÉDÉ DE PRÉPARATION DE GLYCOLS

(30) Priority: 30.06.2015 EP 15174653
(43) Date of publication of application: 09.05.2018
(73) Proprietor: Shell Internationale Research Maatschappij B.V., 2596 HR The Hague (NL)
(72) Inventor: VAN DER HEIDE, Evert, 1031 HW Amsterdam (NL)
(74) Representative: Shell Legal Services IP
(86) International application number: PCT/EP2016/064965
(87) International publication number: WO 2017/001382

(56) References cited:
- WO-A1-2014/161852
- WO-A1-2015/028398
- WO-A1-2015/055315
- WO-A2-2013/015955
- CN-A- 1 762 938
- US-A1- 2011 046 419

## Description

### Field of the Invention

The present invention relates to a process for the preparation of ethylene and propylene glycols from starch-containing feedstocks.

### Background of the Invention

Ethylene glycol and propylene glycol are valuable materials with a multitude of commercial applications, e.g. as heat transfer media, antifreeze, and precursors to polymers, such as PET. Ethylene and propylene glycols are typically made on an industrial scale by hydrolysis of the corresponding alkylene oxides, which are the oxidation products of ethylene and propylene, produced from fossil fuels.

In recent years, increased efforts have focussed on producing chemicals, including glycols, from renewable feedstocks, such as sugar-based materials. The conversion of sugars to glycols can be seen as an efficient use of the starting materials with the oxygen atoms remaining intact in the desired product.

Current methods for the conversion of saccharides to sugars revolve around a hydrogenation/hydrogenolysis process as described in Angew. Chem. Int. Ed. 2008, 47, 8510-8513.

WO 2015/028398 describes a continuous process for the conversion of a saccharide-containing feedstock into glycols. In this process the saccharide-containing feedstock is contacted in a reactor with a catalyst composition comprising at least two active catalytic components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum and compounds and complexes thereof.

WO2013/015955 discloses a process for generating at least one polyol from a feedstock comprising saccharide. The process involves, contacting, hydrogen, water, and a feedstock comprising saccharide, with a catalyst system to generate an effluent stream comprising at least one polyol and recovering the polyol from the effluent stream

CN1762938 discloses a hydrocracking process in which saccharide or alditol is produced into ethylene glycol, propylene glycol and other lower alcohols. The technological process includes two steps. In the first step, the material inside some solvent is hydroreduced into corresponding alditol at relatively low temperature of 50-150 deg C and relatively low hydrogen pressure of 3.5-7.0 MPa. In the second step, catalyst and alkali promoter are added to perform hydrocracking reaction at temperature 150-250 deg C and hydrogen pressure 7.0-10.5 MPa for 3-5 hr to obtain lower alcohols with ethylene glycol and propylene glycol as main components.

WO2014/161852 discloses a process for the preparation of ethylene glycol and 1, 2-propylene glycol from starting material comprising one or more saccharides, by contacting said starting material with hydrogen in a reactor in the presence of a solvent and a catalyst system with catalytic hydrogenation abilities. The process includes the steps of: i) introducing a first portion of the starting material into the reactor such that the initial concentration of the saccharide in the solvent in the reactor is no more than 2 wt%; ii) allowing at least 90wt% of the saccharide in the first portion of the starting material to react; iii) subsequently adding further portions of starting material to the reactor over time; and removing reaction product from the reactor.

WO2015/055315 discloses a process for producing a low boiling mixture comprising ethylene glycol and propylene glycol from a liquid sugar stream derived from a ligno-cellulosic biomass feedstock. The liquid sugar stream is catalytically converted in the presence of hydrogen to a mixture, which is separated into at least a high boiling mixture, comprising glycerol, and the low boiling mixture.

An important aim in this area is the provision of a process that is high yielding in desirable products, such as mono-ethylene glycol (MEG) and mono-propylene glycol (MPG), and that can be sustained with such yields over time.

However, the present inventors have found that, when using catalysts known in the art, such as those taught in WO 2015/028398, for the hydrogenation/ hydrogenolysis of saccharide-containing feedstock comprising starch, significant catalyst deactivation was found to occur over time.

The handling of saccharide-containing feedstocks comprising starch is also complicated. Starch is insoluble in water and is typically fed as a slurry in water to the reactor. Starch slurries can be fed into atmospheric vessel at solid contents up to 50%. However, to prevent gelation and other handling issues, slurries containing greater than about 20wt% of starch in water into a hot and pressurized reactor are avoided.

It would be advantageous to provide a process for the preparation of glycols from saccharide-containing feedstocks comprising starch in which catalyst deactivation is reduced or avoided. It would also be advantageous to be able to provide a more concentrated feed to the hydrogenation/hydrogenolysis reactor than is typically possible when using a starch in water slurry.

### Summary of the Invention

Accordingly, the present invention provides a process for the production of glycols the steps of:
(a) providing a feedstock comprising starch;
(b) subjecting said feedstock to a hydrolysis reaction in the presence of water to provide a liquid hydrolysis product comprising water and glucose and dimers, trimers and oligomers thereof;
(c) subjecting said liquid hydrolysis product to a series of purification steps, comprising one or more filtration steps and one or more adsorption steps, until a pre-treated feedstock stream having a sulfur content of less than 5 ppmw is produced; and
(d) contacting the pre-treated feedstock stream with hydrogen in the presence of a catalyst composition comprising at least two active catalytic components, said active catalyst components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum, lanthanum, tin or compounds or complexes thereof.

### Detailed Description of the Invention

The present invention concerns hydrogenation/ hydrogenolysis processes for the production of ethylene and propylene glycols, in which the feedstock for said reaction, being a saccharide-containing feedstock comprising starch, is pre-treated in order to reduce the impurity content of said feedstock. The treated feedstock is then subjected to hydrogenation/ hydrogenolysis in the presence of a catalyst composition. The inventive process allows the hydrogenation/ hydrogenolysis reactions to be carried out while minimising any deactivation of the catalyst compositions used therefor.

The present inventors have surprisingly found that the amounts of ethylene and propylene glycols yielded by known hydrogenation/hydrogenolysis processes are reduced over time, due to deactivation of the catalytic compositions. It is postulated, without wishing to be bound by any theory, that such deactivation is, at least in part, caused by the presence of impurities. Such impurities include sulfur, nitrogen, phosphorus, chloride and compounds containing these elements in the saccharide-containing feedstocks, in particular sulfur-containing compounds.

The saccharide-containing feedstock for said process comprises starch. It may also comprise one or more further saccharide selected from the group consisting of monosaccharides, disaccharides, oligosaccharides and polysaccharides. Examples of other suitable polysaccharides include cellulose, hemicelluloses, glycogen, chitin and mixtures thereof.

Starch is a polysaccharide comprising a large number of glucose units joined by glycosidic bonds. The glucose units are present as amylose molecules, which are typically linear, and amylopectin molecules, which are branched and present in both crystalline and amorphous forms. Amylose is formed inside amylopectin by amylopectin-bound 'granule bound starch synthase' (GBSS). Such material and other impurities are, therefore, bound within a starch granule and cannot be removed by simple washing steps.

The saccharide-containing feedstock comprising starch may be derived from grains such as corn, wheat, millet, oats, rye, sorghum, barley or buckwheat, from rice, from pulses such as soybean, pea, chickpea or lentil, from bananas and/or from root vegetables such as potato, yam, sweet potato, cassava and sugar beet, or any combinations thereof. A preferred source of saccharide-containing feedstock comprising starch is corn.

In the process of the present invention, the saccharide-containing feedstock comprising starch is subjected to a hydrolysis reaction in the presence of water to provide a liquid hydrolysis product comprising water and glucose and dimers, trimers and oligomers thereof. Any suitable hydrolysis process, e.g. acid-catalysed hydrolysis, may be applied. In a preferred embodiment, enzymatic hydrolysis is used.

A number of pre-hydrolysis steps may be applied to the initial feedstock. These include, but are not limited to sizing, drying, blending, grinding, washing, dewatering, solids removal, steeping, milling, steaming and pre-heating.

Enzymatic hydrolysis may typically be carried out in the presence of one or more amylase enzymes. Suitable temperatures are in the range of from 60 to 120°C, preferably in the range of from 70 to 100°C, more preferably in the range of from 75 to 90°C. In a preferred embodiment, the saccharide-containing feedstock comprising starch is provided to the hydrolysis reaction in water. Preferably, the saccharide-containing feedstock comprising starch makes up at least 20wt%, more preferably at least 30wt%, preferably at most 50wt% of the feed to the hydrolysis reaction.

The hydrolysis step results in a liquid hydrolysis product. Said liquid hydrolysis product comprises glucose and dimers, trimers and oligomers thereof in water.

The liquid hydrolysis product is then subjected to a series of purification steps to produce a pre-treated feedstock stream. Said purification steps comprise one or more filtration steps and one or more adsorption steps and may also comprise other suitable steps for removing impurities from a liquid stream. For example, treatment with active carbon and/or treatment with ion exchange resins may be used.

In one preferred embodiment, the liquid hydrolysis product is filtered, then subjected to treatment with active carbon and then subsequently passed through an ion exchange resin bed. Suitable ion exchange resins for use in such a process include cation exchange resins and anion exchange resins. In a preferred embodiment, a cation exchange resin and an anion exchange resin are used sequentially.

Suitable commercially available ion exchange resins include those comprising polyacrylate or styrenedivinylbenzene copolymers as polymeric backbones. Resins with silica-based polymeric backbones, such as polysiloxanes, and resins incorporating vinylpyridine monomers in their polymeric backbones may also be used. Commercially available anion exchange resins suitable for the process of the present invention include, but are not limited to, Lewatit 500 KR (Lewatit is a trade mark), Amberlite IRA-900, Amberlite IRA-458 (Amberlite is a trade mark), Amberjet 4200, Amberjet 4400 (Amber jet is a trade mark), DOWEX 1x16 (DOWEX is a trade mark), Reillex HPQ (Reillex is a trade mark), Marathon-A, Marathon-MSA (Marathon is a trade mark) and DELOXAN AMP (DELOXAN is a trade mark).

Suitable cation exchange resins may be of the sulfonic type. Commercially available examples are those known by the trademarks AMBERLYST 15, AMBERJET 1500H, AMBERJET 1200H,DOWEX MSC-1, DOWEX 50W, DIANON SK1B, LEWATIT VP OC 1812, LEWATIT S 100 MB and LEWATIT S 100 G1.

The one or more filtration steps and one or more adsorption steps continue until the pre-treated feedstock stream has a concentration of sulfur of no more than 5 ppmw, preferably no more than 1 ppmw, more preferably no more than 0.5 ppmw on the basis of the total stream.

The one or more filtration steps and one or more adsorption steps continue until the pre-treated feedstock stream preferably has a concentration of nitrogen of no more than 150 ppmw, preferably no more than 30 ppmw, more preferably no more than 10 ppmw on the basis of the total stream.

The one or more filtration steps and one or more adsorption steps continue until the pre-treated feedstock stream preferably has a concentration of phosphorus of no more than 25 ppmw, preferably no more than 5 ppmw, more preferably no more than 2 ppmw on the basis of the total stream.

The one or more filtration steps and one or more adsorption steps continue until the pre-treated feedstock stream preferably has a concentration of chloride of no more than 5 ppmw, preferably no more than 1 ppmw, more preferably no more than 0.5 ppmw on the basis of the total stream.

The pre-treated feedstock stream is contacted with hydrogen in the presence of a catalyst composition comprising at least two active catalytic components, said active catalyst components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum, lanthanum, tin and compounds and complexes thereof.

The present invention has the added advantage over many prior art methods of providing a liquid feedstock to this reaction. The liquid is easier to handle than a slurry and can be fed at much higher concentrations of saccharide in water to the reactor.

Preferably, the treated feedstock stream is contacted with hydrogen also in the presence of a solvent. The solvent may be water or a C₁ to C₆ alcohol or polyalcohol or mixtures thereof. Preferred C₁ to C₆ alcohols include methanol, ethanol, 1-propanol and iso-propanol. Polyalcohols of use include glycols, particularly products of the hydrogenation/ hydrogenolysis reaction, glycerol, erythritol, threitol, sorbitol and mixtures thereof. Preferably, the solvent is water. Further solvent may also be added to the reactor or reactors in a separate feed stream or may be added to the treated feedstock stream before it enters the reactor. Said solvent is also suitably water or a C₁ to C₆ alcohol or polyalcohols or mixtures thereof. Preferred C₁ to C₆ alcohols include methanol, ethanol, 1-propanol and iso-propanol. Polyalcohols of use include glycols, particularly products of the hydrogenation/ hydrogenolysis reaction, glycerol, erythritol, threitol, sorbitol and mixtures thereof. Preferably, both solvents are the same. More preferably, both solvents comprise water. Most preferably, both solvents are water.

The treated feedstock stream may be contacted with hydrogen in the presence of a catalyst composition in one or more reactors in parallel or in series.

The catalyst composition and the components contained therein may be heterogeneous or homogeneous with respect to the solvent or solvents present in the reactors during the process of the present invention.

The catalyst composition may also contain both heterogeneous and homogeneous components.

Depending on the physical state of the catalyst composition and any components contained therein, they may be preloaded into the reactors or, if they are in liquid form or present as a solution or slurry in a solvent, they may be fed into the reactor as required in a continuous or discontinuous manner during the process of the present invention.

The catalyst composition comprises at least two active catalytic components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum, lanthanum, tin or compounds or complexes thereof.

Preferably, the first active catalyst component consists of one or more of the group selected from iron, cobalt, nickel, ruthenium, rhodium, palladium, iridium and platinum. This component may be present in the elemental form or as a compound. It is also suitable that this component is present in chemical combination with one or more other ingredients in the catalyst system. It is required that the first active catalyst component has catalytic hydrogenation capabilities and it is capable of catalysing the hydrogenation of material present in the reactor.

Preferably, the second active catalyst component comprises of one or more compound, complex or elemental material comprising tungsten, molybdenum, lanthanum or tin. More preferably, the second active catalyst component comprises one or more material selected from the list consisting of tungstic acid, molybdic acid, ammonium tungstate, ammonium metatungstate, ammonium paratungstate, tungstate compounds comprising at least one Group I or II element, metatungstate compounds comprising at least one Group I or II element, paratungstate compounds comprising at least one Group I or II element, heteropoly compounds of tungsten, heteropoly compounds of molybdenum, tungsten oxides, molybdenum oxides and combinations thereof. The metal component is suitably in a form other than a carbide, nitride, or phosphide. Preferably, the second active catalyst component comprises one or more compound, complex or elemental material selected from those containing tungsten or molybdenum.

Preferably, at least one of the active catalyst components is supported on a solid support. In this embodiment, any other active catalyst component may be present in either heterogeneous or homogeneous form. Said any other active catalyst component may also be supported on a solid support. In one embodiment of the invention, the first active catalyst component is supported on one solid support and the second active catalyst component is supported on a second solid support which may comprise the same or different material. In another embodiment, both active catalyst components are supported on one solid support.

The solid supports may be in the form of a powder or in the form of regular or irregular shapes such as spheres, extrudates, pills, pellets, tablets, monolithic structures. Alternatively, the solid supports may be present as surface coatings, for example on the surfaces of tubes or heat exchangers. Suitable solid support materials are those known to the skilled person and include, but are not limited to aluminas, silicas, zirconium oxide, magnesium oxide, zinc oxide, titanium oxide, carbon, activated carbon, zeolites, clays, silica alumina and mixtures thereof.

Suitably, the weight ratio of the first active catalyst component to the second active catalyst component is in the range of from 0.02:1 to 3000:1, preferably in the range of from 0.1:1 to 100:1, on the basis of the weight of metal present in each component. The weight ratio of the active catalyst components may be varied between the first and second reactors and it may be advantageous to alter the composition of the catalyst systems between the reactors to suit the different feed streams provided to each reactor.

The weight ratio of the first active catalyst component (based on the amount of metal in said component) to sugar is suitably in the range of from 1:100 to 1:1000. The weight ratio of the second active catalyst component (based on the amount of metal in said component) to sugar is suitably in the range of from 1:10 to 1:100.

If more than one reactor is used in series, a catalyst composition may optionally be present in the second and any subsequent reactors. If a catalyst composition is present in the second and any subsequent reactor, the catalyst composition used in each of the reactors may be the same or different. Suitably reaction conditions, particularly temperature and pressure, can be varied between the reactors if more than one reactor is used.

The reaction temperature at which the treated feedstock stream is contacted with hydrogen in the presence of the catalyst composition described herein is suitably at least 130°C, preferably at least 150°C, more preferably at least 170°C, most preferably at least 190°C. The temperature in the reactor is suitably at most 300 °C, preferably at most 280°C, more preferably at most 270°C, even more preferably at most 250°C. Preferably, the reactor is heated to a temperature within these limits before addition of any starting material and is maintained at such a temperature as the reaction proceeds.

The pressure in the reactor or reactors in which the treated feedstock stream is contacted with hydrogen in the presence of the catalyst composition described herein is suitably at least 1 MPa, preferably at least 2 MPa, more preferably at least 3 MPa. The pressure in the reactor is suitably at most 15 MPa, preferably at most 12 MPa, more preferably at most 10 MPa, most preferably at most 8 MPa. Preferably, the reactor is pressurised to a pressure within these limits by addition of hydrogen before addition of any starting material and is maintained at such a pressure as the reaction proceeds through on-going addition of hydrogen.

It may be advantageous to vary the conditions, e.g. temperature and pressure, between the first and any subsequent reactors. This can lead to a more tailored process to suit the different constituents of the feeds provided to each reactor.

The process of the present invention takes place in the presence of hydrogen. Preferably, the process of the present reaction takes place in the absence of air or oxygen. In order to achieve this, it is preferable that the atmosphere in the reactor be evacuated and replaced an inert gas, such as nitrogen, and then with hydrogen repeatedly, after loading of any initial reactor contents, before the reaction starts.

Suitable reactors to be used in the process of the present invention include stirred tank reactors, slurry reactors, ebullated bed reactors, jet flow reactors, mechanically agitated reactors, bubble columns, such as slurry bubble columns and external recycle loop reactors. The use of these reactors allows dilution of the reaction feedstock and intermediates to an extent that provides high degrees of selectivity to the desired glycol product (mainly ethylene and propylene glycols), such as by effective back-mixing.

The residence time in the reactor is suitably at least 1 minute, preferably at least 2 minutes, more preferably at least 5 minutes. Suitably the residence time in the reactor is no more than 5 hours, preferably no more than 2 hours, more preferably no more than 1 hour.
The present invention is further illustrated in the following Examples.

### Examples

### Example 1 (inventive)

A 100 ml Hastelloy C22 autoclave (Premex), equipped with a hollow-shaft gas stirrer, was loaded with 2.5 g Raney Nickel 2800 (Aldrich). A constant liquid volume of 50 ml liquid hold-up was maintained by level control. A feed stream composed of 9.3 %w (defined as glucose equivalence) corn syrup (Golden Barrel, DE-42, commercially available, sulfur content < 3 ppmw S, 5.3 ppm Cl), 3800 ppmw sodium metatungstate (Aldrich), 2300 ppmw NaHCO3 (Aldrich) as a buffer, all dissolved in water, was dosed at a constant flow rate of 44.4 ml/hr. Corn syrup of this type is produced by a hydrolysis reaction and subsequent purification steps (filtration and adsorption). This is clearly indicated by the impurities contents determined. Hydrogen was fed at a rate of 3 liter STP/hr. The temperature and total pressure were maintained at 230° C and 100 barg, respectively and a stirring rate of 1300 rpm was applied. The product stream was analysed by gas chromatography, applying a CPSil-5 column. The yields of the main products monoethylene glycol (MEG), monopropylene glycol (MPG), hydroxyacetone (HA), 1,2-butanediol (1,2-BDO) and 1-hydroxy-2-butanone (1H2BO) are given in Table 1. Yields are defined as weight product recovered divided by weight of glucose times 100%. Ignoring Time = 0 hours, the average MEG yield is 33.7 %w/w and constant without significant decline in yield during time.

### Example 2 (not of the invention)

130 mg Raney Nickel 2800 (Aldrich) and 74.92 ml demineralized water were loaded in a 250 ml Hastelloy C22 autoclave (Premex), equipped with a hollow-shaft gas stirrer. The autoclave was closed, the gascap was replaced by nitrogen and then hydrogen. The autoclave was then pressurised to 35 barg and heated to 195° C in 45 min. Then 24.71 ml of 4.056 w/w% glucose (Merck) solution, containing 50 mg sodium metatungstate (Aldrich) was fed at a rate of 5 ml/min by means of an HPLC pump (LabAlliance, Serie III 301SFT01). The final pressure was adjusted to 80 barg and the mixture was stirred at 1200 rpm for 75 min. The reactor was cooled to 30° C, depressurized and discharged. The product sample was filtered over a 0.45 micron filter and analysed by gas chromatography, applying a CPSil-5 column. The yields of the main products monoethylene glycol (MEG), monopropylene glycol (MPG), hydroxyacetone (HA), 1,2-butanediol (1,2-BDO) and 1-hydroxy-2-butanone (1H2BO) are given in Table 3. Yields are defined as weight product recovered divided by weight of glucose times 100%.

### Example 3 (Comparative)

An experiment comparable to Example 2 (comparative) was executed, with quantities indicated in Table 2, except that 4.7 mg methionine (Merck), corresponding to 10 ppmw S basis total liquid after addition, was added prior to closing the autoclave. The yields of the main products are given in Table 3. Severe catalyst deactivation is apparent, as lower glycol yields are low and yields of the corresponding hydroxyl-ketone intermediates are higher.

### Example 4 (not of the invention)

A subsequent experiment was executed, comparable to Example 2 (comparative), with quantities as given in Table 2, to validate the performance of the autoclave after exposure to sulfur in Example 3 (comparative). The yields of the main products are given in Table 3, indicating a slightly lower yield compared to Example 2 (comparative), but within experimental error. Slight deterioration of the product yield is apparent, relative to Example 2 (comparative), possibly due to the effect of some residual sulfur, although experimental error could also explain part of the deviation.

### Example 5 (comparative)

An experiment was executed as given in Example 2 (comparative), with quantities as given in Table 2, except that 3.8 mg cysteine (Amresco) in total, corresponding to 10 ppmw S basis total liquid after addition, was added together with the glucose solution. The yields of the main products are given in Table 3. Severe catalyst deactivation is apparent, given lower glycol yields and higher yields of the corresponding hydroxyl-ketones.

### Example 6 (comparative)

An experiment was executed as given in Example 2 (comparative), with quantities as given in Table 2, except that 4.7 mg methionine (Merck) in total, corresponding to 10 ppmw S basis total liquid after addition, was added together with the glucose solution. The yields of the main products are given in Table 3. Severe catalyst deactivation is apparent, given lower glycol yields and higher yields of the corresponding hydroxyl-ketones.

### Example 7 (not of the invention)

An experiment was executed as given in Example 2 (comparative), with quantities as given in Table 2, to validate the performance of the autoclave after exposure to sulfur in Examples 5 and 6 (comparative). The yields of the main products are given in Table 3, indicating a slightly lower yield compared to Example 2 (comparative), but within experimental error. No deterioration of the product yield is apparent, relative to Example 4 (comparative).

In summary, the catalyst system applied in the Comparative Examples 3, 5 and 6 is deactivated, resulting in product yields below 10%w MEG (basis 100%w glucose) at a level of 10 ppmw S in about 100 g of liquid in the presence of 111 - 144 mg Raney Ni. This corresponds to a sulfur to catalyst ratio of about 8 gram sulfur per 1 kg Raney Nickel.

**Table 1 - product yields, Example 1**

| **Run time** | **MEG** | **MPG** | **HA** | **1,2BDO** | **1H2BO** | **Total** |
|---|---|---|---|---|---|---|
| (h) | % (w/w) | % (w/w) | % (w/w) | % (w/w) | % (w/w) | % (w/w) |
| 0 | 28.9 | 5.2 | 0.1 | 2.3 | 0.1 | 36.7 |
| 3 | 33.7 | 5.3 | 0.1 | 3.2 | 0.1 | 42.4 |
| 6 | 33.9 | 5.4 | 0.1 | 3.6 | 0.1 | 43.1 |
| 9 | 31.9 | 5.7 | 0.1 | 4.2 | 0.2 | 42.1 |
| 12 | 33.6 | 6.2 | 0.2 | 4.9 | 0.2 | 45.1 |
| 15 | 33.9 | 6.1 | 0.2 | 5.0 | 0.2 | 45.4 |
| 18 | 35.9 | 6.4 | 0.2 | 5.5 | 0.2 | 48.1 |
| 21 | 33.2 | 7.1 | 0.2 | 4.8 | 0.2 | 45.5 |

**Table 2 - Feed input, comparative examples**

| | **W (mg)** | **Ni (mg)** | **Water (g)** | **Amino acid** | **Addition method** | **Glucose conc. (w/w%)** | **Added volume (g)** | **Final glucose conc. (w/w%)** |
|---|---|---|---|---|---|---|---|---|
| **2** | 50 | 130 | 74.92 | - | | 4.06 | 24.71 | 1.01 |
| **3** | 50 | 121 | 74.85 | Methionine | Preloaded | 4.13 | 23.66 | 0.99 |
| **4** | 50 | 131 | 75.87 | - | | 4.13 | 24.98 | 1.06 |
| **5** | 50 | 144 | 74.82 | Cysteine | Fed with glucose | 4.13 | 24.89 | 1.03 |
| **6** | 50 | 111 | 74.98 | Methionine | Fed with glucose | 4.13 | 25.20 | 1.04 |
| **7** | 50 | 120 | 74.74 | - | | 4.13 | 24.86 | 1.03 |

**Table 3 - Product yields, comparative examples**

| | **MEG** % (w/w) | **MPG** % (w/w) | **HA** % (w/w) | **1,2BDO** % (w/w) | **1H2BO** % (w/w) | **Total** % (w/w) |
|---|---|---|---|---|---|---|
| **2** | 31.3 | 3.8 | 3.5 | 2.2 | 4.0 | 44.6 |
| **3** | 2.7 | 0.4 | 8.9 | 0.2 | 8.9 | 21.0 |
| **4** | 27.4 | 1.9 | 1.8 | 1.1 | 2.0 | 34.2 |
| **5** | 9.8 | 0.7 | 7.4 | 0.4 | 7.2 | 25.5 |
| **6** | 2.8 | 0.4 | 8.5 | 0.2 | 8.7 | 20.6 |
| **7** | 29.5 | 2.0 | 1.9 | 1.1 | 2.2 | 36.6 |

The Examples clearly demonstrate the levels of yield expected in the conversion of glucose to MEG and other glycols. This yield is rapidly affected by the presence of the sort of amino acid-bound sulfur present in starch. Example 1 (of the invention) demonstrates that the process of the present invention allows the conversion of starch-based feedstocks to MEG and other glycols without the catalyst deactivation observed when high levels of sulfur impurities are present.

## Claims

1. A process for the production of glycols the steps of:
(a) providing a feedstock comprising starch;
(b) subjecting said feedstock to a hydrolysis reaction in the presence of water to provide a liquid hydrolysis product comprising water and glucose and dimers, trimers and oligomers thereof;
(c) subjecting said liquid hydrolysis product to a series of purification steps, comprising one or more filtration steps and one or more adsorption steps, until a pre-treated feedstock stream having a sulfur content of less than 5 ppmw is produced; and
(d) contacting the pre-treated feedstock stream with hydrogen in the presence of a catalyst composition comprising at least two active catalytic components, said active catalyst components comprising, as a first active catalyst component, one or more materials selected from transition metals from groups 8, 9 or 10 or compounds thereof, with catalytic hydrogenation capabilities; and, as a second active catalyst component, one or more materials selected from tungsten, molybdenum, lanthanum, tin or compounds or complexes thereof.

2. A process as claimed in Claim 1, wherein the hydrolysis reaction comprises an enzymatic hydrolysis reaction.

3. A process as claimed in Claim 2, wherein the enzymatic hydrolysis reaction is carried out in the presence of one or more amylase at a temperature in the range of from 60 to 120°C.

4. A process as claimed in any one of Claims 1 to 3, wherein the adsorption steps comprise treatment with active carbon.

5. A process as claimed in any one of Claims 1 to 4, wherein the adsorption steps comprise treatment with one or more ion exchange resin, selected from cation exchange resins and anion exchange resins.

6. A process as claimed in any one of Claims 1 to 5, wherein the one or more filtration steps and one or more adsorption steps continue until the pre-treated feedstock stream has a phosphorus content of less than 25 ppmw.

7. A process as claimed in any one of Claims 1 to 6, wherein the one or more filtration steps and one or more adsorption steps continue until the pre-treated feedstock stream has a nitrogen content of less than 150 ppmw.

8. A process as claimed in any one of Claims 1 to 7, wherein the one or more filtration steps and one or more adsorption steps continue until the pre-treated feedstock stream has a chloride content of less than 5 ppmw.

9. A process as claimed in any one or Claims 1 to 8, wherein the glycols comprise monoethylene and monopropylene glycols.

## Patentansprüche

1. Verfahren zur Herstellung von Glykolen, mit den Schritten:
(a) Bereitstellen eines Einsatzmaterials, das Stärke umfasst;
(b) Durchführen einer Hydrolysereaktion in Gegenwart von Wasser an dem Einsatzmaterial, um ein flüssiges Hydrolyseprodukt bereitzustellen, das Wasser und Glucose und Dimere, Trimere und Oligomere davon umfasst;
(c) Durchführen einer Reihe von Reinigungsschritten an dem flüssigen Hydrolyseprodukt, umfassend einen oder mehrere Filtrationsschritte und einen oder mehrere Adsorptionsschritte, bis ein vorbehandelter Einsatzmaterialstrom mit einem Schwefelgehalt von weniger als 5 ppmw erzeugt worden ist; und
(d) Inkontaktbringen des vorbehandelten Einsatzmaterialstroms mit Wasserstoff in Gegenwart einer Katalysatorzusammensetzung, die mindestens zwei aktive katalytische Komponenten umfasst, wobei die aktiven Katalysatorkomponenten als eine erste aktive Katalysatorkomponente ein oder mehrere Materialien, mit katalytischen Hydrierungsfähigkeiten, ausgewählt aus Übergangsmetallen der Gruppen 8, 9 oder 10 oder Verbindungen davonund als eine zweite aktive Katalysatorkomponente ein oder mehrere Materialien, ausgewählt aus Wolfram, Molybdän, Lanthan, Zinn oder Verbindungen oder Komplexe davon, umfassen.

2. Verfahren nach Anspruch 1, wobei die Hydrolysereaktion eine enzymatische Hydrolysereaktion umfasst.

3. Verfahren nach Anspruch 2, wobei die enzymatische Hydrolysereaktion in Gegenwart einer oder mehrerer Amylasen bei einer Temperatur im Bereich von 60 bis 120 °C durchgeführt wird.

4. Verfahren nach einem der Ansprüche 1 bis 3, wobei die Adsorptionsschritte eine Behandlung mit Aktivkohle umfassen.

5. Verfahren nach einem der Ansprüche 1 bis 4, wobei die Adsorptionsschritte die Behandlung mit einem oder mehreren Ionenaustauscherharzen, ausgewählt aus Kationenaustauscherharzen und Anionenaustauscherharzen, umfassen.

6. Verfahren nach einem der Ansprüche 1 bis 5, wobei der eine oder die mehreren Filtrationsschritte und der eine oder die mehreren Adsorptionsschritte fortgesetzt werden, bis der vorbehandelte Einsatzmaterialstrom einen Phosphorgehalt von weniger als 25 ppmw aufweist.

7. Verfahren nach einem der Ansprüche 1 bis 6, wobei der eine oder die mehreren Filtrationsschritte und der eine oder die mehreren Adsorptionsschritte fortgesetzt werden, bis der vorbehandelte Einsatzmaterialstrom einen Stickstoffgehalt von weniger als 150 ppmw aufweist.

8. Verfahren nach einem der Ansprüche 1 bis 7, wobei der eine oder die mehreren Filtrationsschritte und der eine oder die mehreren Adsorptionsschritte fortgesetzt werden, bis der vorbehandelte Einsatzmaterialstrom einen Chloridgehalt von weniger als 5 ppmw aufweist.

9. Verfahren nach einem oder mehreren der Ansprüche 1 bis 8, wobei die Glykole Monoethylen- und Monopropylenglykole umfassen.

## Revendications

1. Procédé de préparation de glycols comprenant les étapes de :
(a) fourniture d'une charge d'alimentation comprenant de l'amidon ;
(b) soumission de ladite charge d'alimentation à une réaction d'hydrolyse en présence d'eau pour fournir un produit d'hydrolyse liquide comprenant de l'eau et du glucose et des dimères, des trimères et des oligomères de ceux-ci ;
(c) soumission dudit produit d'hydrolyse liquide à une série d'étapes de purification, comprenant une ou plusieurs étapes de filtration et une ou plusieurs étapes d'adsorption, jusqu'à ce qu'un écoulement de charge d'alimentation prétraité ayant une teneur en soufre inférieure à 5 ppm en poids soit produit ; et
(d) mise en contact de l'écoulement de charge d'alimentation prétraité avec de l'hydrogène en présence d'une composition de catalyseur comprenant au moins deux composants catalytiques actifs, lesdits composants catalytiques actifs comprenant, comme premier composant catalytique actif, une ou plusieurs matières choisies parmi les métaux de transition des groupes 8, 9 ou 10 ou des composés de ceux-ci, ayant des capacités d'hydrogénation catalytique ; et, comme second composant catalytique actif, une ou plusieurs matières choisies parmi le tungstène, le molybdène, le lanthane, l'étain ou des composés ou complexes de ceux-ci.

2. Procédé selon la revendication 1, dans lequel la réaction d'hydrolyse comprend une réaction d'hydrolyse enzymatique.

3. Procédé selon la revendication 2, dans lequel la réaction d'hydrolyse enzymatique est réalisée en présence d'une ou de plusieurs amylases à une température comprise entre 60 et 120 °C.

4. Procédé selon l'une quelconque des revendications 1 à 3, dans lequel les étapes d'adsorption comprennent un traitement avec du charbon actif.

5. Procédé selon l'une quelconque des revendications 1 à 4, dans lequel les étapes d'adsorption comprennent un traitement avec une ou plusieurs résines échangeuses d'ions, choisies parmi les résines échangeuses de cations et les résines échangeuses d'anions.

6. Procédé selon l'une quelconque des revendications 1 à 5, dans lequel les une ou plusieurs étapes de filtration et une ou plusieurs étapes d'adsorption se poursuivent jusqu'à ce que l'écoulement de charge d'alimentation prétraité ait une teneur en phosphore inférieure à 25 ppm en poids.

7. Procédé selon l'une quelconque des revendications 1 à 6, dans lequel les une ou plusieurs étapes de filtration et une ou plusieurs étapes d'adsorption se poursuivent jusqu'à ce que l'écoulement de charge d'alimentation prétraité ait une teneur en azote inférieure à 150 ppm en poids.

8. Procédé selon l'une quelconque des revendications 1 à 7, dans lequel les une ou plusieurs étapes de filtration et une ou plusieurs étapes d'adsorption se poursuivent jusqu'à ce que l'écoulement de charge d'alimentation prétraité ait une teneur en chlorure inférieure à 5 ppm en poids.

9. Procédé selon l'une quelconque des revendications 1 à 8, dans lequel les glycols comprennent des monoéthylèneglycols et des monopropylèneglycols.
